(19) Europäisches Patentamt
European Patent Office
Office européen des brevets

(11) Veröffentlichungsnummer: **0 221 014 A1**

(12) # EUROPÄISCHE PATENTANMELDUNG

(21) Anmeldenummer: **86810431.6**

(22) Anmeldetag: **01.10.86**

(51) Int. Cl.⁴: **C 07 D 239/26**, C 07 D 241/12, A 01 N 43/54, A 01 N 43/60

(30) Priorität: **01.10.85 CH 4244/85**

(43) Veröffentlichungstag der Anmeldung: **06.05.87 Patentblatt 87/19**

(84) Benannte Vertragsstaaten: **AT BE CH DE ES FR GB GR IT LI LU NL SE**

(71) Anmelder: **CIBA-GEIGY AG, Klybeckstrasse 141, CH-4002 Basel (CH)**

(72) Erfinder: **Ackermann, Peter, Dr., Hangelimattweg 113, CH-4148 Pfeffingen (CH)**
Erfinder: **Tobler, Hans, Dr., Baselmattweg 157, CH-4123 Allschwil (CH)**
Erfinder: **Nyfeler, Robert, Dr., Bärenfelserstrasse 8, CH-4057 Basel (CH)**

(54) **Phenoxyverbindungen als Mikrobizide.**

(57) Pyrimidinyl- resp. Pyrazinylderivate der Formel I

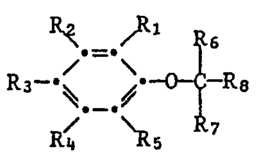

(I),

worin

**$R_1$ bis $R_5$** unabhängig voneinander Wasserstoff, Halogen, $C_1$-$C_6$-Alkyl, $C_1$-$C_6$-Halogenalkyl, $C_1$-$C_6$-Alkoxy, $C_1$-$C_6$-Halogenalkoxy, Cyano, $C_1$-$C_6$-Alkoxycarbonyl oder Phenyl,

**$R_6$ und $R_7$** unabhängig voneinander Wasserstoff, $C_1$-$C_6$-Alkyl, $C_3$-$C_6$-Alkenyl oder $C_3$-$C_6$-Alkinyl sowie gegebenenfalls durch Halogen, $C_1$-$C_6$-Alkyl, $C_1$-$C_6$-Halogenalkyl, $C_1$-$C_6$-Alkoxy oder $C_1$-$C_6$-Halogenalkoxy substituiertes Phenyl oder Benzyl bedeuten, und

**$R_8$** 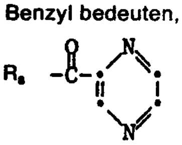 , 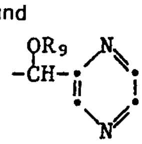 , 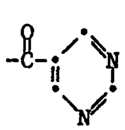 , oder 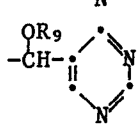 und

$R_9$ Wasserstoff, $C_1$-$C_6$-Alkyl, $C_3$-$C_6$-Alkenyl, $C_3$-$C_6$-Alkinyl oder gegebenenfalls durch Halogen, $C_1$-$C_6$-Alkyl, $C_1$-$C_6$-Halogenalkyl, $C_1$-$C_6$-Alkoxy, $C_1$-$C_6$-Halogenalkoxy substituiertes Benzyl bedeuten, stellen wertvolle Mikrobizide dar. Es werden Verfahren zur Herstellung dieser Verbindungen und ihre Verwendung in der Schädlingsbekämpfung, vor allem gegen phytopathogene Bakterien und Pilze beschrieben.

EP 0 221 014 A1

0221014

CIBA-GEIGY AG
Basel (Schweiz)

5-15518/+

Phenoxyverbindungen als Mikrobizide

Die vorliegende Erfindung betrifft Phenoxiäthanon- resp. Phenoxiäthanol-Derivate sowie deren Säureadditionssalze und Metallkomplexe, Verfahren zu ihrer Herstellung und ihre Verwendung in der Schädlingsbekämpfung. Die neuen Verbindungen haben die Formel I

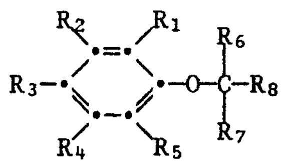

(I),

worin

$R_1$ bis $R_5$ unabhängig voneinander Wasserstoff, Halogen, $C_1$-$C_6$-Alkyl, $C_1$-$C_6$-Halogenalkyl, $C_1$-$C_6$-Alkoxy, $C_1$-$C_6$-Halogenalkoxy, Cyano, $C_1$-$C_6$-Alkoxycarbonyl oder Phenyl,

$R_6$ und $R_7$ unabhängig voneinander Wasserstoff, $C_1$-$C_6$-Alkyl, $C_3$-$C_6$-Alkenyl oder $C_3$-$C_6$-Alkinyl sowie Phenyl oder Benzyl, deren aromatischer Ring unsubstituiert oder durch Halogen, $C_1$-$C_6$-Alkyl, $C_1$-$C_6$-Halogenalkyl, $C_1$-$C_6$-Alkoxy oder $C_1$-$C_6$-Halogenalkoxy substituiert sein kann,

$R_8$ 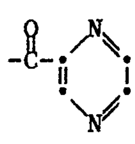 , 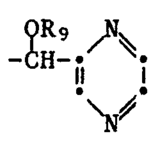 , 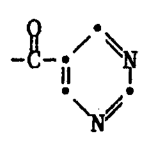 , oder 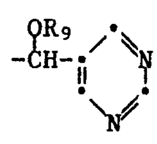 und

$R_9$ Wasserstoff, $C_1$-$C_6$-Alkyl, $C_3$-$C_6$-Alkenyl, $C_3$-$C_6$-Alkinyl oder Benzyl, das unsubstituiert oder im Ring durch Halogen, $C_1$-$C_6$-Alkyl, $C_1$-$C_6$-Halogenalkyl, $C_1$-$C_6$-Alkoxy, $C_1$-$C_6$-Halogenalkoxy substituiert sein kann, bedeuten, unter Einschluss der Säureadditionssalze und Metallkomplexe.

Wie ersichtlich, betrifft die vorliegende Erfindung Phenoxyäthanonrespektive Phenoxyäthanol-Derivate, bei denen der Substituent $R_8$ einen Sechsring-Heterocyclus mit 2 N-Atomen enthält.

Die Verbindungen der Formel I bilden demgemäss je nach Bedeutung des Substituenten $R_8$ zwei Reihen von Präparaten, die Pyrazinyl-2-Derivate und die Pyrimidinyl-5-Derivate.

Die bei $R_1$ bis $R_9$ in Frage kommenden Alkyl-, Halogenalkyl-, Alkoxy-, Halogenalkoxy-, Alkenyl- und Alkinylgruppen können geradkettig oder verzweigt sein.

Unter dem Begriff Alkyl selbst oder als Bestandteil eines anderen Substituenten wie Alkoxy, Halogenalkyl, Halogenalkoxy etc., sind je nach Zahl der angegebenen Kohlenstoffatome beispielsweise die folgenden Gruppen zu verstehen: Methyl, Aethyl, Propyl, Butyl, Pentyl, Hexyl sowie ihre Isomeren, wie z.B. Isopropyl, Isobutyl, tert.-Butyl, Isopentyl usw. Halogen und Halo stehen stellvertretend für Fluor, Chlor, Brom oder Jod. Haloalkyl steht somit für einen einfach bis perhalogenierten Alkylrest, wie z.B. $CHCl_2$, $CH_2F$, $CCl_3$, $CH_2Cl$; $CHFCH_3$ $CH_2CH_2Br$, $CF_2CF_3$, $C_2Cl_5$, $CH_2Br$, CHBrCl usw., vorzugsweise für $CF_3$ und $CHF_2$. Die vorstehenden Beispiele gelten sinngemäss für Halogenalkoxy. Alkenyl steht z.B. für Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw., vorzugsweise Propargyl.

Die vorliegende Erfindung betrifft somit die freien Verbindungen der Formel I, deren Säureadditionssalze und deren Metallkomplexe, wobei die freien Verbindungen bevorzugt sind.

Beispiele salzbildender Säuren sind unter den anorganischen Säuren Halogenwasserstoffsäuren wie Fluorwasserstoffsäure, Chlorwasserstoffsäure, Bromwasserstoffsäure oder Jodwasserstoffsäure sowie ferner Schwefelsäure, Phosphorsäure, phosphorige Säure, Salpetersäure, und unter den organischen Säuren Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chlor, Mangan, Eisen, Kobalt, Nickel, Zirkonium, Kupfer, Zink, Silber, usw. Bevorzugt sind die Nebengruppen-Elemente der 4. Periode. Die Metalle können dabei in die verschiedenen ihnen zukommenden Wertigkeit vorliegen. Die Metallkomplexe der Formel I können ein-oder mehrkernig auftreten, d.h. sie können ein oder mehrere organische Molekülanteile als Liganden enthalten. Komplexe mit den Metallen, Kupfer, Zink, Mangan, Zinn und Zirkonium sind bevorzugt.

Bevorzugt sind Verbindungen der Formel I, worin

$R_1$ bis $R_5$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy sind, oder einer dieser Substituenten auch Phenyl sein kann,

$R_6$ und $R_7$ unabhängig voneinander Wasserstoff, $C_1$-$C_6$-Alkyl, $C_3$-$C_5$-Alkenyl, $C_3$-$C_5$-Alkinyl, Phenyl oder Benzyl bedeuten, die im Ring durch Halogen substituiert sein können, und

$R_9$ Wasserstoff, $C_1$-$C_4$-Alkyl, $C_3$-$C_4$-Alkenyl, $C_3$-$C_4$-Alkinyl, Benzyl oder Halobenzyl darstellt, während $R_8$ die für Formel I angegebene Bedeutung hat. (Verbindungsgruppe Ia).

Eine der wichtigen Untergruppen der Verbindungsgruppe I-a sind jene Pyrazinyl-2-Derivate der Formel I mit

$R_8$ = CO-Pyrazinyl(2) oder -CH($OR_9$)-Pyrazinyl(2),
worin
$R_1$ = Wasserstoff, Methyl, Aethyl oder Halogen,
$R_2$ = Wasserstoff,
$R_3$ = Wasserstoff, Halogen, $C_1$-$C_3$-Halogenalkyl, $C_1$-$C_3$-Halogenalkoxy, CN oder Phenyl,
$R_4$ = Wasserstoff oder Halogen,
$R_5$ = Wasserstoff oder Halogen,
$R_6$ = Wasserstoff, $C_1$-$C_4$-Alkyl, $C_3$-$C_5$-Alkenyl, $C_3$-$C_5$-Alkinyl, Phenyl oder Benzyl, deren aromatischer Ring auch halogensubstituiert sein kann, und
$R_7$ = Wasserstoff oder $C_1$-$C_3$-Alkyl,
$R_9$ = Wasserstoff, $C_1$-$C_4$-Alkyl, Allyl, Propargyl, oder Benzyl
bedeuten. (Verbindungsgruppe I-b)

Innerhalb der Verbindungsgruppe I-b sind jene Verbindungen bevorzugt, worin
$R_1$ = Wasserstoff, Fluor oder Chlor,
$R_3$ = Fluor, Chlor oder Brom, $CF_3$, $CF_3$-O- oder $CHF_2$-O-, und $R_2$, $R_4$ und $R_5$ Wasserstoff bedeuten, $R_6$ Wasserstoff, $C_2$-$C_4$-Alkyl, Allyl, Propargyl, Phenyl Chlorphenyl,
$R_7$ Wasserstoff oder Methyl und
$R_9$ = Wasserstoff, Methyl, Aethyl, Isopropyl, Allyl oder Benzyl
darstellen. (Verbindungsgruppe I-c).
In der Verbindungsgruppe I-c sind jene Vertreter besonders bevorzugt, worin $R_6$ für einen der aliphatischen Substituenten steht.

Eine der wichtigen Untergruppen der Verbindungsgruppe I-a sind jene Pyrimidinyl-5-Derivate der Formel I mit
$R_8$ = CO-Pyrimidinyl(5) oder -CH($OR_9$)-Pyrimidinyl(5),
worin
$R_1$ = Wasserstoff, Methyl, Aethyl oder Halogen,
$R_2$ = Wasserstoff,
$R_3$ = Wasserstoff, Halogen, $C_1$-$C_3$-Halogenalkyl, $C_1$-$C_3$-Halogenalkoxy, CN oder Phenyl,
$R_4$ = Wasserstoff oder Halogen,

$R_5$ = Wasserstoff oder Halogen,

$R_6$ = Wasserstoff, oder $C_1$-$C_4$-Alkyl, $C_3$-$C_5$-Alkenyl, $C_3$-$C_5$-Alkinyl, Phenyl oder Benzyl, deren aromatischer Ring auch halogensubstituiert sein kann,

$R_7$ = Wasserstoff oder $C_1$-$C_3$-Alkyl, und

$R_9$ = Wasserstoff, $C_1$-$C_4$-Alkyl, Allyl, Propargyl, oder Benzyl

bedeuten. (Verbindungsgruppe Id)

Innerhalb der Verbindungsgruppe Id sind jene Verbindungen bevorzugt, worin

$R_1$ = Wasserstoff, Fluor oder Chlor,

$R_3$ = Fluor, Chlor oder Brom, $CF_3$, $CF_3$-O- oder $CHF_2$-O-, und $R_2$, $R_4$ und $R_5$ Wasserstoff bedeuten, $R_6$ Wasserstoff, $C_2$-$C_4$-Alkyl, Allyl, Propargyl, Phenyl, Chlorphenyl, und $R_7$ Wasserstoff oder Methyl und $R_9$ = Wasserstoff, Methyl, Aethyl, Isopropyl, Allyl oder Benzyl darstellen. (Verbindungsgruppe Ie)

In der Verbindungsgruppe I-e sind jene Vertreter besonders bevorzugt, worin $R_6$ für einen aliphatischen Substituenten steht.

Unter den bevorzugten Beispielen von Verbindungen der Formel I sind folgende Vertreter:

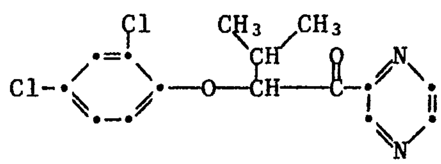

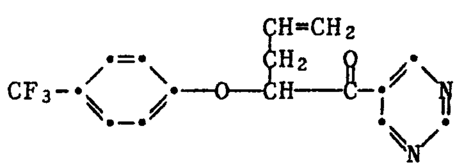

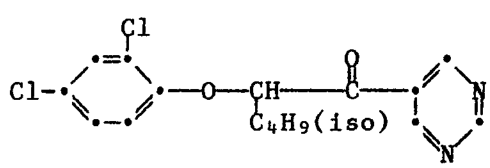

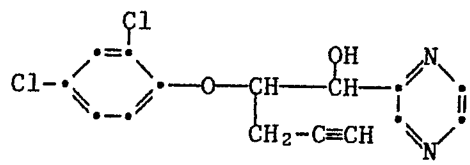

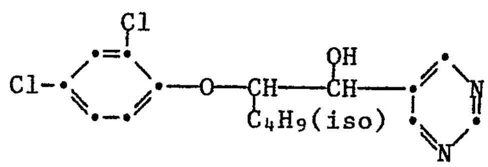

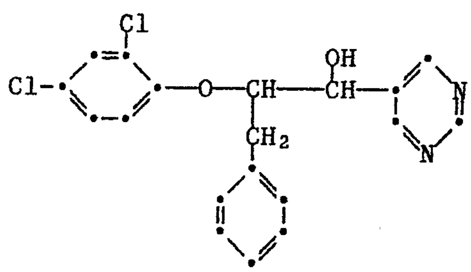

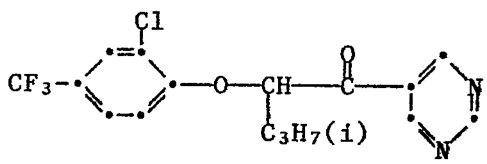

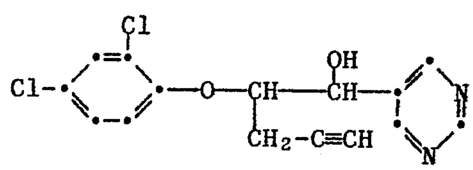

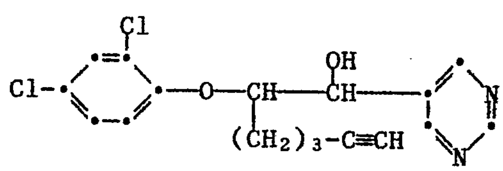

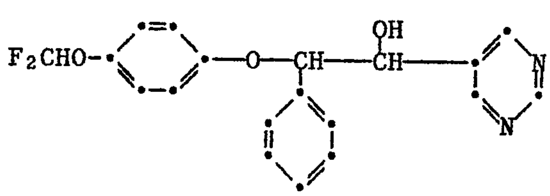

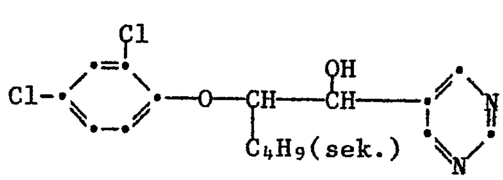

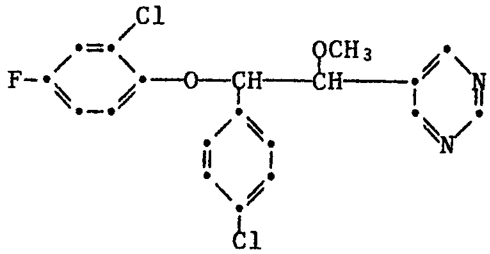

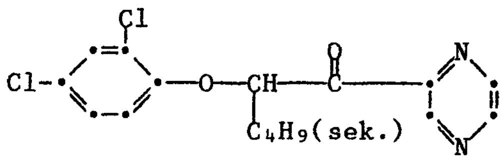

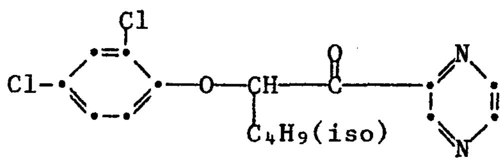

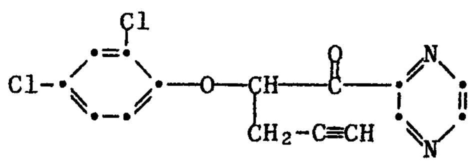
.

sowie die Verbindungen

1-(5-Pyrimidinyl)-2-(2,4-dichlorphenoxy)-2-methyl-butan-1-on,

1-(2-Pyrazinyl)-2-(3,4-dichlorphenoxy)-pentan-1-on,

1-(2-Pyrazinyl)-2-(2,4-dichlorphenoxy)-butan-1-ol,

1-(5-Pyrimidinyl)-2-(2,4-dichlorphenoxy)-hexan-1-on,

1-(5-Pyrimidinyl)-2-(4-phenyl-phenoxy)-butan-1-on,

1-(2-Pyrazinyl)-2-(4-chlorphenoxy)-3-methyl-pentan-1-on,

1-(5-Pyrimidinyl)-2-(2-methyl-4-chlorphenoxy)-butan-1-ol,

1-(2-Pyrazinyl)-2-(2,4-dichlorphenoxy)-3-methyl-pentan-1-on.

Die erfindungsgemässen Verbindungen der

Formel I, worin $R_8$ 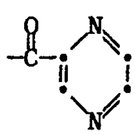 oder 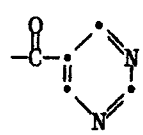

bedeutet, können z.B. erhalten werden, in dem man a) einen Ester der Formel II

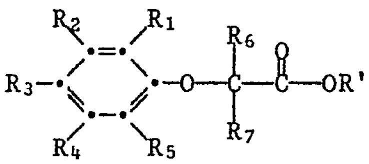

(II)

mit einem Halogenid der Formeln III oder IIIa

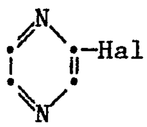

(III)

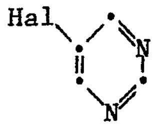

(IIIa)

in Gegenwart eines Metallierungsmittels wie Mg (z.B. in Form einer Grignard-Verbindung) oder wie Butyllithium umsetzt oder b) eine Verbindung der Formeln IV oder IVa

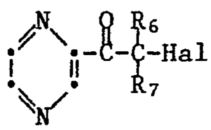

(IV)

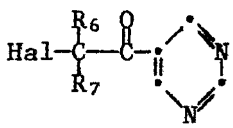

(IVa)

mit einem Phenol der Formel V

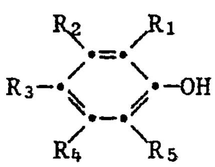

(V)

in Gegenwart einer Base reagieren lässt, wobei $R_1$ bis $R_7$ die für Formel I gegebene Bedeutung haben und Hal Halogen, bevorzugt Chlor, Brom oder Jod, bedeutet, während R' für $C_1$-$C_4$-Alkyl, $C_3$-$C_4$-Alkenyl oder für Phenyl oder Benzyl steht, die unsubstituiert oder durch Alkyl, Alkoxy, Halogen, $NO_2$, CN ringsubstituiert sein können.

Als Basen kommen bei dem Verfahren b) insbesondere tertiäre Amine, wie Trialkylamine und Pyridin, ferner Hydroxide, Oxide, Carbonate und Bicarbonate von Alkali- und Erdalkalimetallen sowie Alkalimetallalkoholate, wie z.B. Kalium-t.butylat oder Alkalihydride in Betracht.

Sollen Verbindungen der Formel I hergestellt werden, bei denen $R_6$ und/oder $R_7$ eine andere Bedeutung als Wasserstoff haben, so ist es zuweilen vorteilhaft, in einem ersten Reaktionsschritt eine Verbindung der Formel IV bzw. IVa, bei der $R_6 = R_7 =$ Wasserstoff bedeutet, mit dem gewünschten Phenol der Formel V zur Reaktion zu bringen, und dann in einem Folgeschritt in die entstandene Verbindung der Formel I den gewünschten Substituenten $R_6$ und/oder $R_7$ an der aktivierten $CH_2$-Gruppe in Form einer Verbindung

$R_6$-hal (VIa) oder $R_7$-hal (VIb)

einzuführen, wobei hal Halogen, bevorzugt Brom oder Jod, bedeutet. Diese Substitution wird in Gegenwart starker Basen wie Na-Methylat oder bevorzugt K-tert.Butylat durchgeführt.

Das Verfahren a) wird entweder in Abwesenheit oder bevorzugt in Anwesenheit eines Lösungs- oder Verdünnungsmittels und bei einer Temperatur im Bereich von -130° bis +50°C, vorzugsweise bei -130° bis +20°C, durchgeführt.

Das Verfahren b) wird gleichfalls bevorzugt in einem Lösungsmittel bei Temperaturen von -50° bis +150°C, bevorzugt 0° bis 120°C, durchgeführt.

Als Lösungs- oder Verdünnungsmittel kommen inerte, hydroxylgruppenfreie Vertreter in Frage, z.B. Aether und ätherartige Verbindungen, wie Diäthyläther, Diisopropyläther, Dioxan, 1,2-Dimethoxyäthan und Tetrahydrofuran; Amide, wie N,N-dialkylierte Carbonsäureamide vom Typ Dimethylformamid; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton oder Methyläthylketon.

Die nach den oben stehenden Verfahren hergestellten Ketone der Formel I können nach bekannten Methoden durch Hydrierung und, sofern gewünscht, anschliessende Reaktion mit einer Verbindung der Formel VII

R"Hal (VII)

in Verbindungen der Formel I überführt werden,

worin $R_8$ 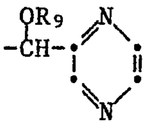 oder 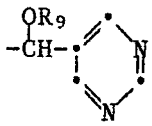 und $R_9$ Wasserstoff, $C_1$-$C_6$-Alkyl,

$C_3$-$C_6$-Alkenyl, $C_3$-$C_6$-Alkinyl oder Benzyl, das im Ring durch Halogen, $C_1$-$C_6$-Alkyl, $C_1$-$C_6$-Halogenalkyl, $C_1$-$C_6$-Alkoxy, $C_1$-$C_6$-Halogenalkoxy substituiert sein kann, bedeuten. In der Formel VII steht R" für $C_1$-$C_6$-Alkyl, $C_3$-$C_6$-Alkenyl, $C_3$-$C_6$-Alkinyl oder gegebenenfalls durch Halogen, $C_1$-$C_6$-Alkyl, $C_1$-$C_6$-Halogenalkyl, $C_1$-$C_6$-Alkoxy, $C_1$-$C_6$-Halogenalkoxy substituiertes Benzyl. Verbindungen der Formeln II bis VII sind bekannt oder können nach bekannten Verfahren hergestellt werden.

Verbindungen der Formel I können, falls sie ein asymmetrisches Kohlenstoffatom besitzen, in zwei enantiomeren Formen vorliegen. Im allgemeinen entsteht bei der Herstellung dieser Substanzen ein Gemisch beider Enantiomerer; dieses lässt sich auf bekannte Weise in die reinen optischen Antipoden aufspalten. Verbindungen der Formel I mit zwei Asymmetrie-Zentren liegen auch als Gemische von diastereomeren Formen vor, die mittels physikalischer Methoden getrennt werden können. Die vorliegende Erfindung betrifft alle reinen Enantiomeren bzw. Diastereomeren und deren Gemische untereinander.

Verbindungen der Formel I besitzen ein für praktische Bedürfnisse sehr günstiges Mikrobizid-Spektrum gegen phytopathogene Pilze und Bakterien. Sie haben sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe der Formel I sind gegen die den folgenden Klassen angehörenden phytopathogenen Pilze wirksam: Fungi imperfecti (z.B. Botrytis, Helminthosporium, Fusarium Septoria, Cercospora und Alternaria); Basidiomyceten (z.B. die Gattungen Hemileia, Rhizoctonia, Puccinia); insbesondere wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden. Die Verbindungen der Formel I besitzen teilweise auch pflanzenregulatorische Wirkung und können in höheren Dosierungen zur Zügelung des übermässigen vegetativen Wachstums an Kulturpflanzen verwendet werden.

Die Erfindung betrifft weiterhin mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken,

Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend

gewählt. Günstige Aufwendmengen liegen im allgemeinen bei 50 g bis 5 kg Aktivsubstanz (AS) je ha, bevorzugt 100 g bis 2 kg AS/ha, insbesondere bei 150 g bis 600 g AS/ha.

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethylenglykolmonomethyl-ether, Ketone, wie Cyclohexanon, stark polare Lösungsmittel wie N-Methyl-2-pyrrolidon, Dimethylsulfoxid oder Dimethylformamid, sowie gegebenenfalls epoxydierte Pflanzenöle oder Sojaöl; oder Wasser.

Als feste Trägerstoffe, z.B. für Stäubemittel und dispergierbare Pulver, lassen sich Calcit, Talkum, Kaolin, Montmorillonit oder Attapulgit, hochdisperse Kieselsäure oder saugfähige Polymerisate verwenden. Als gekörnte, adsorptive Granulatträger kommen Bimsstein, Ziegelbruch, Sepiolit oder Bentonit, als nicht-sorptive Träger z.B. Calcit oder Dolomit in Frage. Es können auch zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood New Jersey, 1980
Sisley and Wood, "Encyclopedia of Surface Active Agents", Chemical Publishing Co., Inc. New York, 1980.

Besonders vorteilhafte, applikationsfördernde Zuschlagstoffe sind ferner natürliche oder synthetische Phospholipide aus der Reihe der Kephaline und Lecithine, wie z.B. Phosphatidylethanolamin, Phosphatidylserin, Phosphatidylglycerin, Lysolecithin.

Die agrochemischen Zubereitungen enthalten in der Regel 0,1 bis 99 %, insbesondere 0,1 bis 95 % Wirkstoff der Formel I, 99,9 bis 1 %, insbesondere 99,9 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze, wie Stabilisatoren, Entschäumer, Viskositätsregulatoren, Bindemittel, Haftmittel sowie Dünger oder andere Wirkstoffe zur Erzielung spezieller Effekte enthalten.

Beispiel H-1:
Herstellung von 1-(2-Pyrazinyl)-2-methyl-2-(4-chlorphenoxy)-propan-1-on

Zu einer Lösung von 20 g 2-Methyl-2-(4-chlorphenoxy)-propionsäureäthylester und 21,7 g Jodpyrazin in 350 ml Diäthyläther werden bei -70°C während 20 Minuten 80 ml einer 1,6 N Lösung von Butyllithium in Hexan zugetropft. Das Reaktionsgemisch wird 1 Stunde bei -70°C gerührt, dann mit 10 Tropfen einer gesättigten Ammoniumchloridlösung versetzt und auf 20°C erwärmt. Anschliessend wird die organische Phase mit gesättigter Ammoniumchloridlösung und 3mal mit je 100 ml Sole (konz. NaCl-Lösung) gewaschen, über $MgSO_4$ getrocknet und eingeengt.

Nach der Reinigung des Rohproduktes mittels Säulenchromatographie an Kieselgel (Eluiermittel: Toluol/Essigester 95:5) erhält man die Verbindung Nr. 1.04, Smp. 84-85°C.

Beispiel H-2:

Herstellung von 1-(2-Pyrazinyl)-2-methyl-2-(4-chlorphenoxy)-propan-1-ol

Zu einer Lösung von 0,12 g Natriumborhydrid in 1 ml Wasser wird bei 0°C während 30 Minuten eine Lösung von 2,8 g 1-(2-Pyrazinyl)-2-methyl-2-(4-chlorphenoxy)-propan-1-on in 7 ml Tetrahydrofuran zugetropft. Das Reaktionsgemisch wird 2 Stunden bei 20°C gerührt und anschliessend mit Aether verdünnt. Die organische Phase wird mit 15 ml gesättigter Ammoniumchloridlösung und 3 mal mit je 15 ml Sole gewaschen, über $MgSO_4$ getrocknet und eingeengt. Nach der Reinigung des Rohproduktes mittels Säulenchromatographie an Kieselgel (Eluiermittel: Toluol/Essigester 4:1) erhält man die Verbindung Nr. 3.17, $n_D^{23°} = 1,5697$.

Beispiel H-3:

Herstellung der Verbindung 1-(2-Pyrazinyl)-1-methoxy-2-(2,4-dichlorphenoxy)-butan der Formel

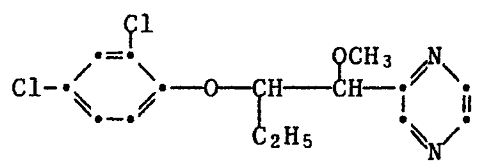

Zu einer Lösung von 1,3 g 1-(2-Pyrazinyl)-2-(2,4-dichlorphenoxy)-butanol und 0,1 g Natriumhydrid in 8 ml Tetrahydrofuran wird bei 10°C eine Lösung von 0,6 g Methyljodid in 2 ml Tetrahydrofuran getropft. Das Reaktionsgemisch wird vier Stunden bei 20°C gerührt. Zur Aufarbeitung werden 0,5 ml gesättigte $NH_4Cl$-Lösung in 50 ml Essigester zugegeben. Die organische Phase wird mit zweimal 20 ml Sole gewaschen, über $MgSO_4$ getrocknet und eingeengt. Das Rohprodukt wird an Kieselgel mit Toluol/Essigester (4:1) als Eluiermittel chromatographiert. Man erhält die Verbindung Nr. 3.05, $n_D^{25°} = 1,5593$.

Beispiel H-4:

Herstellung der Verbindung 1-(5-Pyrimidinyl)-2-(2,4-dichlorphenoxy)-2-phenyl-äthan-1-on der Formel

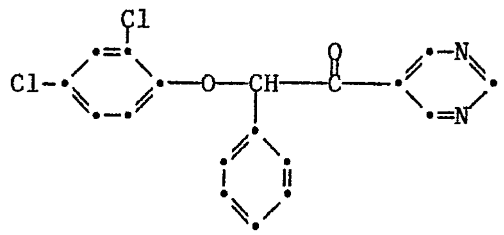

In eine Lösung von 22 g 2-(2,4-Dichlorphenoxy)-2-phenyl-essigsäure-methylester der Formel

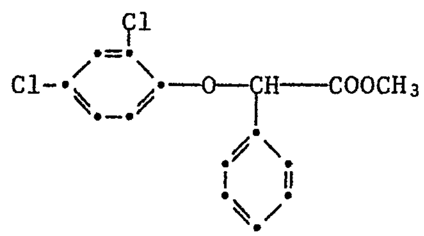

und 12,4 g 5-Brompyrimidin in 140 ml Diäthyläther, 100 ml Tetrahydrofuran und 100 ml Pentan werden innerhalb von 75 Minuten 46,4 ml Butyllithium (1,6 molar in Hexan) bei -110°C zugetropft. Das Reaktionsgemisch wird 4 Stunden bei -80°C gerührt und innerhalb von 8 Stunden auf 20°C erwärmt. Nach der Zugabe von 0,5 ml gesättiger $NH_4Cl$-Lösung wird mit Diäthyläther versetzt. Die organische Phase wird mit Sole gewaschen, über $MgSO_4$ getrocknet und eingeengt. Das Rohprodukt wird an Kieselgel mit Toluol/Hexan (9:1) als Eluiermittel chromatographiert. Man erhält die Verbindung Nr. 2.20, $n_D^{25°} = 1,6081$.

Auf diese oder analoge Weise werden folgende Verbindungen hergestellt:

Tabelle 1

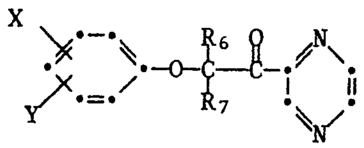

Me = Methyl   Et = Aethyl

| Verb. Nr. | X | Y | R6 | R7 | Physik. Konstante |
|---|---|---|---|---|---|
| 1.01 | 4-Cl | 2-Cl | H | H | Smp.129-130°C |
| 1.02 | 4-Cl | H | H | H | |
| 1.03 | 4-Cl | 2-Cl | Me | H | |
| 1.04 | 4-Cl | H | Me | Me | Smp. 84-85° |
| 1.05 | 4-Cl | 2-Cl | Me | Me | $n_D^{23}$ = 1,5779 |
| 1.06 | 4-Cl | H | Et | H | |
| 1.07 | 4-Cl | 2-Cl | Et | H | $n_D^{25}$ = 1,5727 |
| 1.08 | 4-Cl | 2-Cl | Et | Me | $n_D^{23}$ = 1,5741 |
| 1.09 | 4-Cl | H | Et | Me | $n_D^{23}$ = 1,5658 |
| 1.10 | 4-F | H | Et | H | $n_D^{24}$ = 1,5458 |
| 1.11 | 4-Br | H | Et | H | $n_D^{24}$ = 1,5850 |
| 1.12 | 4-$CH_3$ | H | Et | H | |
| 1.13 | 4-Cl | 2-Cl | n-Propyl | H | $n_D^{23}$ = 1,5684 |
| 1.14 | 4-Cl | 2-Cl | n-Butyl | H | $n_D^{29}$ = 1,5609 |
| 1.15 | 4-Cl | 2-Cl | Isopropyl | H | |
| 1.16 | 4-Cl | 2-Cl | tert.Butyl | H | Smp. 75-78° |
| 1.17 | 4-Cl | 2-Cl | n-Hexyl | H | |
| 1.18 | 4-Cl | 2-Cl | Allyl | H | |
| 1.19 | 4-Cl | 2-Cl | $-CH_2-C{\equiv}CH$ | H | $n_D^{24}$ = 1,5967 |
| 1.20 | 4-Cl | 2-Cl | $-C_6H_5$ | H | Smp.156-158°C |
| 1.21 | 4-Cl | 2-Cl | $-C_6H_4Cl(4)$ | H | Smp. 128-129°C |
| 1.22 | 4-Cl | 2-Cl | $-C_6H_3Cl_2(2,4)$ | H | |

Tabelle 1 (Fortsetzung)

| Verb. Nr. | X | Y | $R_6$ | $R_7$ | Physik. Konstante |
|---|---|---|---|---|---|
| 1.23 | 4-Cl | 2-Cl | $-CH_2C_6H_5$ | H | |
| 1.24 | 4-Cl | 2-Cl | $-CH_2-C_6H_4Cl(4)$ | H | |
| 1.25 | 4-Cl | H | $-C_6H_5$ | H | Smp. 153-154°C |
| 1.26 | $4-OCHF_2$ | H | $-C_6H_5$ | H | |
| 1.27 | $4-OC_3H_7$ | H | Et | H | |
| 1.28 | $3-CF_3$ | H | Et | H | |
| 1.29 | $4-CF_3$ | H | Isopropyl | H | |
| 1.30 | 4-CN | H | Isopropyl | H | |
| 1.31 | $4-CO_2CH_3$ | H | Isopropyl | H | |
| 1.32 | $4-C_6H_5$ | H | Et | H | $n_D^{24}$ = 1,6040 |
| 1.33 | $4-C_6H_5$ | H | $-C_6H_5$ | H | |
| 1.34 | 4-Cl | 2-Cl | sek.Butyl | H | halbfest |
| 1.35 | 4-Cl | H | n-Butyl | H | $n_D^{29}$ = 1,5528 |
| 1.36 | 4-Cl | H | $-C_6H_4Cl(4)$ | H | Smp. 99-100°C |
| 1.37 | 4-Cl | 2-Cl | Isobutyl | H | Smp. 95-96°C |
| 1.38 | 4-Cl | H | $-C_6H_4Cl(2)$ | H | $n_D^{24}$ = 1,6149 |
| 1.39 | 4-Cl | 2-Cl | $-C_6H_3Cl_2(2.6)$ | H | Smp. 143-145°C |
| 1.40 | 4-Cl | 2-Cl | $-C_6H_4Cl(2)$ | H | $n_D^{24}$ = 1,6155 |
| 1.41 | 4-Cl | H | n-Propyl | H | $n_D^{24}$ = 1,5631 |
| 1.42 | $4-CF_3$ | H | Et | H | Smp. 54-56°C |
| 1.43 | 4-F | 2-Cl | Et | H | $n_D^{25}$ = 1,5532 |
| 1.44 | 4-Cl | H | $-C_6H_3Cl_2(2,6)$ | H | Smp. 119-120°C |
| 1.45 | 4-Cl | 3-Cl | n-Propyl | H | Smp. 67-68°C |

Tabelle 1 (Fortsetzung)

| Verb. Nr. | X | Y | $R_6$ | $R_7$ | Physik. Konstante |
|---|---|---|---|---|---|
| 1.46 | 4-Cl | 2-$CH_3$ | $CH_3$ | H | $n_D^{26}$ = 1,5705 |
| 1.47 | 4-Cl | H | Isobutyl | H | $n_D^{24}$ = 1,5535 |
| 1.48 | 4-Cl | H | sek.Butyl | H | halbfest |
| 1.49 | 4-Cl | 3-$CH_3$ | Et | H | $n_D^{23}$ = 1,5640 |
| 1.50 | 4-$C_6H_5$ | H | H | H | Smp. 138-139°C |
| 1.51 | 4-Cl | 2-$CH_3$ | n-Propyl | H | $n_D^{25}$ = 1,5558 |

Auf analoge Weise werden auch folgende Verbindungen hergestellt:

Tabelle 2

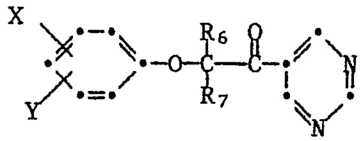

Me = Methyl  Et = Aethyl

| Verb. Nr. | X | Y | $R_6$ | $R_7$ | Physik. Konstante |
|---|---|---|---|---|---|
| 2.01 | 4-Cl | 2-Cl | H | H | |
| 2.02 | 4-Cl | H | H | H | |
| 2.03 | 4-Cl | 2-Cl | Me | H | |
| 2.04 | 4-Cl | H | Me | Me | |
| 2.05 | 4-Cl | 2-Cl | Me | Me | Smp. 106-107°C |
| 2.06 | 4-Cl | H | Et | H | |
| 2.07 | 4-Cl | 2-Cl | Et | H | $n_D^{29}$ = 1,5741 |
| 2.08 | 4-Cl | 2-Cl | Et | Me | Smp. 99-100°C |
| 2.09 | 4-Cl | H | Et | Me | |
| 2.10 | 4-F | H | Et | H | $n_D^{24}$ = 1,5430 |
| 2.11 | 4-Br | H | Et | H | $n_D^{24}$ = 1,5820 |
| 2.12 | 4-Me | H | Et | H | |
| 2.13 | 4-Cl | 2-Cl | n-Propyl | H | $n_D^{25}$ = 1,5657 |
| 2.14 | 4-Cl | 2-Cl | n-Butyl | H | Smp. 78-80,5°C |
| 2.15 | 4-Cl | 2-Cl | Isopropyl | H | $n_D^{24}$ = 1,5676 |
| 2.16 | 4-Cl | 2-Cl | tert.Butyl | H | |
| 2.17 | 4-Cl | 2-Cl | n-Hexyl | H | |
| 2.18 | 4-Cl | 2-Cl | Allyl | H | |
| 2.19 | 4-Cl | 2-Cl | $-CH_2-C\equiv CH$ | H | |
| 2.20 | 4-Cl | 2-Cl | $-C_6H_5$ | H | $n_D^{25}$ = 1,6081 |
| 2.21 | 4-Cl | 2-Cl | $-C_6H_4Cl(4)$ | H | Smp.127-129°C (Zers.) |
| 2.22 | 4-Cl | 2-Cl | $-C_6H_4Cl(4)$ | H | |
| 2.23 | 4-Cl | 2-Cl | $-CH_2C_6H_5$ | H | |

Tabelle 2 (Fortsetzung)

| Verb. Nr. | X | Y | $R_6$ | $R_7$ | Physik. Konstante |
|---|---|---|---|---|---|
| 2.24 | 4-Cl | 2-Cl | $-CH_2-C_6H_4Cl(4)$ | H | |
| 2.25 | $4-OCH_3$ | H | $-C_6H_5$ | H | |
| 2.26 | $4-OCHF_2$ | H | $-C_6H_5$ | H | |
| 2.27 | $4-OC_3H_7$ | H | Et | H | |
| 2.28 | $3-CF_3$ | H | Et | H | |
| 2.29 | $4-CF_3$ | H | Isopropyl | H | |
| 2.30 | 4-CN | H | Isopropyl | H | |
| 2.31 | $4-CO_2CH_3$ | H | Isopropyl | H | |
| 2.32 | $4-C_6H_5$ | H | Et | H | $n_D^{24}$ = 1,6089 |
| 2.33 | $4-C_6H_5$ | H | $-C_6H_5$ | H | |
| 2.34 | 4-Cl | 2-Cl | sek.Butyl | H | |
| 2.35 | 4-Cl | H | n-Butyl | H | $n_D^{29}$ = 1,5519 |
| 2.36 | 4-F | 2-Cl | Et | H | $n_D^{29}$ = 1,5510 |
| 2.37 | 4-Cl | 2-Cl | Isobutyl | H | Smp. 70-71°C |
| 2.38 | 4-Cl | 2-Cl | $-C_6H_4Cl(2)$ | H | Smp. 84-86°C |
| 2.39 | 4-Cl | H | $-C_6H_4Cl(2)$ | H | $n_D^{24}$ = 1,6113 |
| 2.40 | 4-Cl | H | n-Propyl | H | $n_D^{24}$ = 1,5592 |
| 2.41 | 4-Cl | H | $-C_6H_5$ | H | Smp.116-117°C |
| 2.42 | $4-CF_3$ | H | Et | H | Smp. 59-61°C |
| 2.43 | 4-Cl | 3-Cl | n-Propyl | H | $n_D^{24}$ = 1,5709 |
| 2.44 | 4-Cl | H | $C_6H_3Cl_2(2,6)$ | H | Smp. 96-97°C |
| 2.45 | 4-Cl | 2-Cl | $C_6H_3Cl_2(2,6)$ | H | $n_D^{24}$ = 1,6114 |
| 2.46 | 4-Cl | H | Isobutyl | H | $n_D^{26}$ = 1,5515 |
| 2.47 | 4-Cl | $2-CH_3$ | $CH_3$ | H | Smp. 101-103°C |
| 2.48 | 4-Cl | $2-CH_3$ | Et | H | $n_D^{30}$ = 1,5612 |

Auf analoge Weise werden auch folgende Verbindungen hergestellt:

Tabelle 3

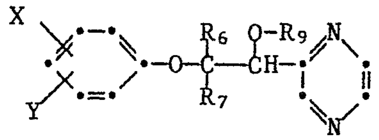

Me = Methyl Et = Aethyl

| Verb. Nr. | X | Y | $R_6$ | $R_7$ | $R_9$ | Physik. Konstante |
|---|---|---|---|---|---|---|
| 3.01 | 4-Cl | 2-Cl | H | H | H | Smp. 6062°C |
| 3.02 | 4-Cl | 2-Cl | H | H | Me | |
| 3.03 | 4-Cl | 2-Cl | Me | H | H | |
| 3.04 | 4-Cl | 2-Cl | Et | H | H | $n_D^{25}$ = 1,5708 |
| 3.05 | 4-Cl | 2-Cl | Et | H | Me | $n_D^{23}$ = 1,5593 |
| 3.06 | 4-Cl | 2-Cl | Et | H | n-Hexyl | |
| 3.07 | 4-Cl | 2-Cl | Et | H | $-CH_2-C \equiv CH$ | $n_D^{25}$ = 1,5624 |
| 3.08 | 4-Cl | 2-Cl | Et | Me | H | $n_D^{23}$ = 1,5732 |
| 3.09 | 4-Cl | 2-Cl | Isopropyl | H | $-(CH_2)_3-C \equiv CH$ | |
| 3.10 | 4-Cl | 2-Cl | n-Propyl | H | $-CH_2C_6H_5$ | |
| 3.11 | 4-Cl | 2-Cl | n-Propyl | H | H | $n_D^{28}$ = 1,5658 |
| 3.12 | 4-Cl | 2-Cl | Me | Me | H | $n_D^{23}$ = 1,5764 |
| 3.13 | 4-Cl | H | $C_6H_5$ | H | Allyl | |
| 3.14 | 4-$C_6H_5$ | H | Isopropyl | H | H | |
| 3.15 | 4-Cl | 2-Cl | $-CH_2-C \equiv CH$ | H | H | |
| 3.16 | 4-Cl | 2-Cl | Allyl | H | H | |
| 3.17 | 4-Cl | H | Me | Me | H | $n_D^{23}$ = 1,5697 |
| 3.18 | 4-Cl | H | Et | Me | H | $n_D^{23}$ = 1,5641 |
| 3.19 | 4-Cl | 2-Cl | $C_6H_5$ | H | H | $n_D^{28}$ = 1,6023 |
| 3.20 | 4-Cl | H | $C_6H_5$ | H | H | Smp.160-161°C |
| 3.21 | 4-Cl | 2-Cl | $C_6H_4Cl(4)$ | H | H | Smp.136-139°C |

Tabelle 3 (Fortsetzung)

| Verb. Nr. | X | Y | $R_6$ | $R_7$ | $R_9$ | Physik. Konstante |
|---|---|---|---|---|---|---|
| 3.22 | 4-Cl | 2-Cl | $-C_6H_5$ | H | $-CH_2C_6H_4Cl(4)$ | |
| 3.23 | 4-Cl | 2-Cl | $-CH_2C_6H_5$ | H | H | |
| 3.24 | 4-Cl | 2-Cl | sek.Butyl | H | H | |
| 3.25 | 4-Cl | H | n-Butyl | H | H | $n_D^{29}$ = 1,5500 |
| 3.26 | 4-Cl | 2-Cl | Isobutyl | H | H | $n_D^{24}$ = 1,5569 |
| 3.27 | 4-Cl | 2-Cl | $-C_6H_4Cl(2)$ | H | H | Smp.138-142°C |
| 3.28 | 4-Cl | H | n-Propyl | H | H | $n_D^{24}$ = 1,5629 |
| 3.29 | 4-F | H | Et | H | H | $n_D^{24}$ = 1,5462 |
| 3.30 | 4-Br | H | Et | H | H | $n_D^{24}$ = 1,5751 |
| 3.31 | 4-Cl | H | $-C_6H_4Cl(4)$ | H | H | Smp. 86-88°C |
| 3.32 | 4-Cl | 2-Cl | n-Butyl | H | H | $n_D^{25}$ = 1,5611 |
| 3.33 | $4-CF_3$ | H | Et | H | H | $n_D^{26}$ = 1,5150 |
| 3.34 | 4-F | 2-Cl | Et | H | H | $n_D^{26}$ = 1,5540 |
| 3.35 | $4-C_6H_5$ | H | Et | H | H | $n_D^{26}$ = 1,6052 |
| 3.36 | 4-Cl | 2-Cl | $-C_6H_3Cl_2(2.6)$ | H | H | wachsartig |
| 3.37 | 4-Cl | H | $-C_6H_4Cl(2)$ | H | H | Smp.104-106°C |
| 3.38 | 4-F | 2-Cl | Et | H | $-CH_2-CH=CH_2$ | $n_D^{24}$ = 1,5342 |
| 3.39 | 4-Cl | 3-Cl | n-Propyl | H | H | $n_D^{24}$ = 1.5629 |
| 3.40 | 4-Cl | H | $C_6H_3Cl_2(2,6)$ | H | H | Smp.120-124°C |

Tabelle 3 (Fortsetzung)

| Verb. Nr. | X | Y | $R_6$ | $R_7$ | $R_9$ | Physik. Konstante |
|---|---|---|---|---|---|---|
| 3.41 | 4-Cl | H | Isobutyl | H | H | halbfest |
| 3.42 | 4-Cl | 2-Cl | Et | H | $-CH_2-CH=CH_2$ | $n_D^{30} = 1,5521$ |
| 3.43 | 4-Cl | $2-CH_3$ | $CH_3$ | H | H | $n_D^{30} = 1,5709$ |
| 3.44 | 4-Cl | 2-Cl | Et | H | Et | $n_D^{27} = 1,5482$ |
| 3.45 | 4-Cl | $2-CH_3$ | Et | H | H | $n_D^{25} = 1,5647$ |

Auf analoge Weise werden auch folgende Verbindungen hergestellt:

Tabelle 4

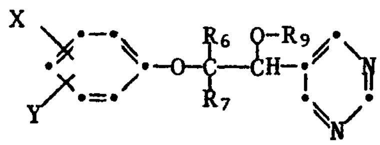

Me = Methyl  Et = Aethyl

| Verb. Nr. | X | Y | $R_6$ | $R_7$ | $R_9$ | Physik. Konstante |
|---|---|---|---|---|---|---|
| 4.01 | 4-Cl | 2-Cl | H | H | H | |
| 4.02 | 4-Cl | 2-Cl | H | H | Me | |
| 4.03 | 4-Cl | 2-Cl | Me | H | H | |
| 4.04 | 4-Cl | 2-Cl | Et | H | H | $n_D^{29}$ = 1,5598 |
| 4.05 | 4-Cl | 2-Cl | Et | H | Me | |
| 4.06 | 4-Cl | 2-Cl | Et | H | n-Hexyl | |
| 4.07 | 4-Cl | 2-Cl | Et | H | $-CH_2-C\equiv CH$ | |
| 4.08 | 4-Cl | 2-Cl | Et | Me | H | |
| 4.09 | 4-Cl | 2-Cl | Isopropyl | H | $-(CH_2)_3-C\equiv CH$ | |
| 4.10 | 4-Cl | 2-Cl | Isopropyl | H | Benzyl | |
| 4.11 | 4-Cl | 2-Cl | Isopropyl | H | H | wachsartig |
| 4.12 | 4-Cl | 2-Cl | Me | Me | H | |
| 4.13 | 4-Cl | H | $C_6H_5$ | H | Allyl | |
| 4.14 | $4-C_6H_5$ | H | Isopropyl | H | H | |
| 4.15 | 4-Cl | 2-Cl | $-CH_2-C\equiv CH$ | H | H | |
| 4.16 | 4-Cl | 2-Cl | Allyl | H | H | |
| 4.17 | 4-Cl | H | Me | Me | H | |
| 4.18 | 4-Cl | H | Et | Me | H | |
| 4.19 | 4-Cl | 2-Cl | $C_6H_5$ | H | H | Smp.174-176°C |
| 4.20 | 4-Cl | H | $C_6H_5$ | H | H | wachsartig |
| 4.21 | 4-Cl | 2-Cl | $C_6H_4Cl(4)$ | H | H | |
| 4.22 | 4-Cl | 2-Cl | $-C_6H_5$ | H | $-CH_2C_6H_4Cl(4)$ | |
| 4.23 | 4-Cl | 2-Cl | Benzyl | H | H | |
| 4.24 | 4-Cl | 2-Cl | sek.Butyl | H | H | |
| 4.25 | 4-Cl | 2-Cl | Isobutyl | H | H | $n_D^{24}$ = 1,5561 |

Tabelle 4 (Fortsetzung)

| Verb. Nr. | X | Y | $R_6$ | $R_7$ | $R_9$ | Physik. Konstante |
|---|---|---|---|---|---|---|
| 4.26 | 4-Cl | 2-Cl | $-C_6H_4Cl(2)$ | H | -Me | $n_D^{24}$ = 1,5779 |
| 4.27 | 4-Cl | 2-Cl | $-C_6H_4Cl(2)$ | H | H | Smp. 79-84°C |
| 4.28 | 4-Cl | H | $-C_6H_4Cl(2)$ | H | H | Smp.115-119°C |
| 4.29 | 4-Cl | 2-Cl | n-Propyl | H | H | $n_D^{24}$ = 1,5645 |
| 4.30 | 4-Cl | H | n-Propyl | H | H | $n_D^{24}$ = 1,5610 |
| 4.31 | 4-F | H | Et | H | H | $n_D^{24}$ = 1,5443 |
| 4.32 | 4-Cl | H | n-Butyl | H | H | $n_D^{24}$ = 1,5538 |
| 4.33 | 4-F | 2-Cl | Et | H | H | $n_D^{24}$ = 1,5527 |
| 4.34 | $4-CF_3$ | H | Et | H | H | $n_D^{24}$ = 1,5140 |
| 4.35 | $4-C_6H_5$ | H | Et | H | H | Smp. 78-80°C |
| 4.36 | 4-Cl | 3-Cl | n-Propyl | H | H | $n_D^{24}$ = 1,5683 |
| 4.37 | 4-Cl | H | $C_6H_3Cl_2(2,6)$ | H | H | Smp.188-192,5°C |
| 4.38 | 4-Cl | H | Isobutyl | H | H | Smp. 80-82°C |
| 4.39 | 4-Cl | $2-CH_3$ | Et | H | H | Smp. 84-88°C |
| 4.40 | 4-Cl | $2-CH_3$ | $CH_3$ | H | H | Smp.111-115°C |
| 4.41 | 4-Cl | 2-Cl | n-Propyl | H | Allyl | $n_D^{27}$ = 1,5488 |
| 4.42 | 4-Cl | 2-Cl | n-Propyl | H | -Me | $n_D^{27}$ = 1,5487 |

Formulierungsbeispiele für Wirkstoffe der Formel I

(% = Gewichtsprozent)

| F-1: Emulsions-Konzentrate | a) | b) | c) | d) |
|---|---|---|---|---|
| Wirkstoff gemäss den Tabellen | 10 % | 25 % | 40 % | 50 % |
| Ca-Dodecylbenzolsulfonat | - | 5 % | 8 % | 6 % |
| Ricinusöl-polyäthylenglykoläther (36 Mol AeO) | - | 5 % | - | - |
| Tributylphenol-polyäthylenglykoläther (30 Mol AeO) | - | - | 12 % | 4 % |
| Ricinusölthioxilat | 25 % | - | - | - |
| Cyclohexanon | - | - | 15 % | 20 % |
| Butanol | 15 % | - | - | - |
| Xylolgemisch | - | 65 % | 25 % | 20 % |
| Essigester | 50 % | - | - | - |

Aus solchen Konzentraten können durch Verdünnen mit Wasser Emulsionen jeder gewünschten Konzentration hergestellt werden.

| F-2: Lösungen | a) | b) |
|---|---|---|
| Wirkstoff gemäss den Tabellen | 10 % | 5 % |
| Aethylenglykol-monomethyläther | - | - |
| Polyäthylenglykol (MG 400) | 70 % | - |
| N-Methyl-2-pyrrolidon | 20 % | - |
| Epoxidiertes Kokosnussöl | - | 1 % |
| Benzin (Siedegrenzen 160-190°C) | - | 94 % |

Die Lösungen sind zur Anwendung in Form kleinster Tropfen geeignet.

| F-3: Spritzpulver | a) | b) | c) |
|---|---|---|---|
| Wirkstoff gemäss den Tabellen | 20 % | 50 % | 75 % |
| Na-Ligninsulfonat | 5 % | 5 % | - |
| Na-Laurylsulfat | 3 % | - | 5 % |

| | | | |
|---|---|---|---|
| Na-Diisobutylnaphthalinsulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F-4: Suspensions-Konzentrat

| | |
|---|---|
| Wirkstoff gemäss den Tabellen | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele:

Beispiel B-1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer

Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

b) Systemische Wirkung

Zu Weizenpflanzen werden 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Verbindungen aus den Tabellen zeigen gegen Puccinia-Pilze eine sehr gute Wirkung. Unbehandelte aber infizierte Kontrollpflanzen zeigen einen Puccinia-Befall von 100 %. Die Verbindungen Nr. 1.34, 1.37, 1.45-1.51, 2.08, 2.14, 2.32, 2.43-2.48, 3.04, 3.07, 3.26, 3.38-3.45, 4.25, 4.37-4.42 und andere hemmen den Puccinia-Befall auf 10 % oder weniger.

Beispiel B-2: Wirkung gegen Cercospora arachidicola auf Erdnusspflanzen

Residual-protektive Wirkung

10-15 cm hohe Erdnusspflanzen werden mit einer aus Spritzpulver der Wirksubstanz hergestellten Spritzbrühe (0,006 Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigen Erdnusspflanzen, die mit Wirkstoffen aus den Tabellen behandelt werden, einen stark reduzierten Cercospora-Befall. Die Verbindungen der Formel I, z.B. Nr. 1.16, 1.19, 1.34, 1.37, 1.45, 1.48, 2.43-2.48, 3.07, 3.20, 3.21, 3.38, 3.40, 4.25, 4.37-4.42, verhindern das Auftreten von Flecken fast vollständig (0-10 % Befall).

Beispiel B-3: Wirkung gegen Erysiphae graminis auf Gerste

a) Residual protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen der Formel I zeigen gute Wirkung gegen Erysiphe-Pilze. Unbehandelte, aber infizierte Kontrollpflanzen zeigen einen Erysiphe-Befall von 100 %. Die Verbindungen der Nr. 1.01, 1.04, 1.05, 1.07-1.11, 1.16, 1.25, 1.34, 1.37-1.51, 2.05, 2.07, 2.08, 2.32, 2.35-2.48, 3.01, 3.04, 3.05, 3.07, 3.11, 3.12, 3.20, 3.21, 3.25-3.45, 4.04, 4.19, 4.20, 4.25-4.42 hemmen den Pilzbefall bei Gerste auf 0 bis 5 %.

Beispiel B-4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,006 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt. Verbindungen der Formel I hemmen den Krankheitsbefall auf weniger als 10 %. Unbehandelte aber infizierte Kontrolltriebe werden dagegen 100%ig befallen.

Beispiel B-5: Wirkung gegen Botrytis cinerea auf Bohnen

Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21°C erfolgt die Beurteilung des Pilzbefalls. Die Verbindungen aus der Tabelle hemmen in vielen Fällen die Pilzinfektion sehr stark. Bei einer Konzentration von 0,02 % erweisen sich die Verbindungen Nr. 2.14, 2.32, 3.04, 4.11, 4.39 als voll wirksam. Der Krankheitsbefall liegt bei 0 bis 8 %.

Beispiel B-6: Wirkung gegen Piricularia oryzae auf Reispflanzen

Residual-protektive Wirkung

Reispflanzen werden nach zweiwöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24°C wird der Pilzbefall beurteilt.

Reispflanzen, die mit einer Spritzbrühe behandelt worden sind, die als Aktivsubstanz eine der Verbindungen der Formel I enthält, zeigen im Vergleich zu unbehandelten Kontrollpflanzen (100 % Befall) weniger als 10 % Pilzbefall.

Patentansprüche

1. Ein Phenoxyäthanon- resp. Phenoxäthanolderivat der Formel I

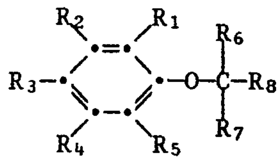 (I),

worin

$R_1$ bis $R_5$ unabhängig voneinander Wasserstoff, Halogen, $C_1$-$C_6$-Alkyl, $C_1$-$C_6$-Halogenalkyl, $C_1$-$C_6$-Alkoxy, $C_1$-$C_6$-Halogenalkoxy, Cyano, $C_1$-$C_6$-Alkoxycarbonyl oder Phenyl,

$R_6$ und $R_7$ unabhängig voneinander Wasserstoff, $C_1$-$C_6$-Alkyl, $C_3$-$C_6$-Alkenyl oder $C_3$-$C_6$-Alkinyl sowie Phenyl oder Benzyl, deren aromatischer Ring unsubstituiert oder durch Halogen, $C_1$-$C_6$-Alkyl, $C_1$-$C_6$-Halogenalkyl, $C_1$-$C_6$-Alkoxy oder $C_1$-$C_6$-Halogenalkoxy substituiert sein kann,

$R_8$ 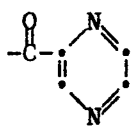 , 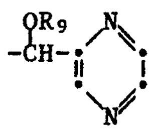 , 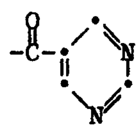 , oder 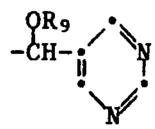 und

$R_9$ Wasserstoff, $C_1$-$C_6$-Alkyl, $C_3$-$C_6$-Alkenyl, $C_3$-$C_6$-Alkinyl oder Benzyl, das unsubstituiert oder im Ring durch Halogen, $C_1$-$C_6$-Alkyl, $C_1$-$C_6$-Halogenalkyl, $C_1$-$C_6$-Alkoxy, $C_1$-$C_6$-Halogenalkoxy substituiert sein kann, bedeuten, unter Einschluss der Säureadditionssalze und Metallkomplexe.

2. Eine Verbindung der Formel I gemäss Anspruch 1, worin

$R_1$ bis $R_5$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy sind, oder einer dieser Substituenten auch Phenyl sein kann,

$R_6$ und $R_7$ unabhängig voneinander Wasserstoff, $C_1$-$C_6$-Alkyl, $C_3$-$C_5$-Alkenyl, $C_3$-$C_5$-Alkinyl, Phenyl oder Benzyl bedeuten, die im Ring durch Halogen substituiert sein können, und

$R_9$ Wasserstoff, $C_1$-$C_4$-Alkyl, $C_3$-$C_4$-Alkenyl, $C_3$-$C_4$-Alkinyl, Benzyl oder Halobenzyl darstellt, während $R_8$ die für Formel I angegebene Bedeutung hat.

3. Pyrazinyl-Derivate der Formel I gemäss Anspruch 2, worin
$R_8$ = CO-Pyrazinyl(2) oder -CH($OR_9$)-Pyrazinyl(2),
darstellt,
$R_1$ = Wasserstoff, Methyl, Aethyl oder Halogen,
$R_2$ = Wasserstoff,
$R_3$ = Wasserstoff, Halogen, $C_1$-$C_3$-Halogenalkyl, $C_1$-$C_3$-Halogenalkoxy, CN oder Phenyl,
$R_4$ = Wasserstoff oder Halogen,
$R_5$ = Wasserstoff oder Halogen,
$R_6$ = Wasserstoff, $C_1$-$C_4$-Alkyl, $C_3$-$C_5$-Alkenyl, $C_3$-$C_5$-Alkinyl, Phenyl oder Benzyl, deren aromatischer Ring auch halogensubstituiert sein kann, und
$R_7$ = Wasserstoff oder $C_1$-$C_3$-Alkyl,
$R_9$ = Wasserstoff, $C_1$-$C_4$-Alkyl, Allyl, Propargyl, oder Benzyl
bedeuten.

4. Pyrazinyl-Derivate gemäss Anspruch 3, worin
$R_1$ = Wasserstoff, Fluor oder Chlor,
$R_3$ = Fluor, Chlor oder Brom, $CF_3$, $CF_3$-O- oder $CHF_2$-O-, und $R_2$, $R_4$ und $R_5$ Wasserstoff bedeuten, $R_6$ Wasserstoff, $C_2$-$C_4$-Alkyl, Allyl, Propargyl, Phenyl Chlorphenyl,
$R_7$ Wasserstoff oder Methyl und
$R_9$ = Wasserstoff, Methyl, Aethyl, Isopropyl, Allyl oder Benzyl
darstellen.

5. Pyrazinyl-Derivate gemäss Anspruch 4, worin $R_6$ für einen der aliphatischen Substituenten steht.

6. Pyrimidinyl-Derivate der Formel I gemäss Anspruch 2, worin
$R_8$ = CO-Pyrimidinyl(5) oder -CH($OR_9$)-Pyrimidinyl(5),
darstellt,

$R_1$ = Wasserstoff, Methyl, Aethyl oder Halogen,

$R_2$ = Wasserstoff,

$R_3$ = Wasserstoff, Halogen, $C_1$-$C_3$-Halogenalkyl, $C_1$-$C_3$-Halogenalkoxy, CN oder Phenyl,

$R_4$ = Wasserstoff oder Halogen,

$R_5$ = Wasserstoff oder Halogen,

$R_6$ = Wasserstoff, oder $C_1$-$C_4$-Alkyl, $C_3$-$C_5$-Alkenyl, $C_3$-$C_5$-Alkinyl, Phenyl oder Benzyl, deren aromatischer Ring auch halogensubstituiert sein kann, sind und

$R_7$ = Wasserstoff oder $C_1$-$C_3$-Alkyl, und

$R_9$ = Wasserstoff, $C_1$-$C_4$-Alkyl, Allyl, Propargyl, oder Benzyl bedeutet.

7. Pyrimidinyl-Derivate gemäss Anspruch 6, worin
$R_1$ = Wasserstoff, Fluor oder Chlor,
$R_3$ = Fluor, Chlor oder Brom, $CF_3$, $CF_3$-O- oder $CHF_2$-O-, und $R_2$, $R_4$ und $R_5$ Wasserstoff bedeuten, $R_6$ Wasserstoff, $C_2$-$C_4$-Alkyl, Allyl, Propargyl, Phenyl, Chlorphenyl, und $R_7$ Wasserstoff oder Methyl und $R_9$ = Wasserstoff, Methyl, Aethyl, Isopropyl, Allyl oder Benzyl darstellen.

8. Pyrimidinyl-Derivate gemäss Anspruch 7, worin $R_6$ für einen der aliphatischen Substituenten steht.

9. 1-(5-Pyrimidinyl)-2-(2,4-dichlorphenoxy)-2-methyl-butan-1-on,
1-(5-Pyrimidinyl)-2-(2,4-dichlorphenoxy)-hexan-1-on,
1-(5-Pyrimidinyl)-2-(4-phenyl-phenoxy)-butan-1-on oder
1-(5-Pyrimidinyl)-2-(2-methyl-4-chlorphenoxy)-butan-1-ol
gemäss Anspruch 6.

10. 1-(2-Pyrazinyl)-2-(3,4-dichlorphenoxy)-pentan-1-on,
1-(2-Pyrazinyl)-2-(2,4-dichlorphenoxy)-butan-1-ol,
1-(2-Pyrazinyl)-2-(4-chlorphenoxy)-3-methyl-pentan-1-on oder
1-(2-Pyrazinyl)-2-(2,4-dichlorphenoxy)-3-methyl-pentan-1-on
gemäss Anspruch 3.

11. Verfahren zur Herstellung von Verbindungen der Formel I gemäss Anspruch 1, dadurch gekennzeichnet, dass man a) einen Ester der Formel II

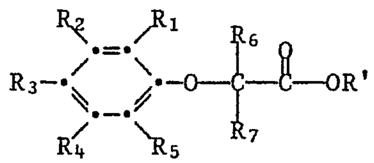

(II)

worin R' für $C_1$-$C_4$-Alkyl, $C_3$-$C_4$-Alkenyl oder Phenyl oder Benzyl steht, die unsubstituiert oder durch Alkyl, Alkoxy, Halogen, $NO_2$, CN im Ring substituiert sein können, mit einem Halogenid der Formeln III oder IIIa

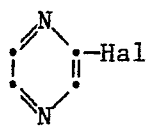

(III)

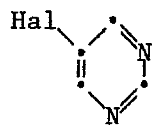

(IIIa)

in Gegenwart eines Metallierungsmittels wie Mg oder wie Butyllithium zur Reaktion bringt, oder b) eine Verbindung der Formeln IV oder IVa

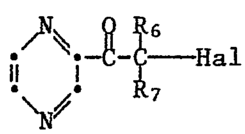

(IV)

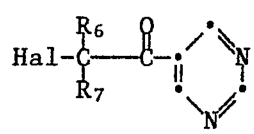

(IVa)

mit einem Phenol der Formel V

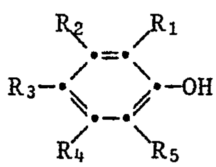

(V)

in Gegenwart einer Base reagieren lässt und die gemäss a) oder b) entstandenen Ketone der Formel I, sofern gewünscht, durch Hydrierung und, sofern gewünscht, anschliessende Reaktion mit einer Verbindung der Formel VII

R"Hal (VII)

in Verbindungen der Formel I überführt

worin $R_8$ 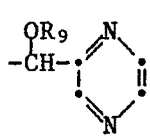 oder 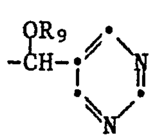 bedeutet und worin

$R_9$ Wasserstoff, $C_1$-$C_6$-Alkyl, $C_3$-$C_6$-Alkenyl, $C_3$-$C_6$-Alkinyl oder Benzyl darstellt, das unsubstituiert ist oder im Ring durch Halogen, $C_1$-$C_6$-Alkyl, $C_1$-$C_6$-Halogenalkyl, $C_1$-$C_6$-Alkoxy, $C_1$-$C_6$-Halogenalkoxy substituiert sein kann, und wobei $R_1$ bis $R_7$ die für die Formel I im Anspruch 1 angegebene Bedeutung haben, während Hal Halogen bedeutet, und R" für $C_1$-$C_6$-Alkyl, $C_3$-$C_6$-Alkenyl, $C_3$-$C_6$-Alkinyl oder Benzyl steht, das unsubstituiert oder im Ring durch Halogen, $C_1$-$C_6$-Alkyl, $C_1$-$C_6$-Halogenalkyl, $C_1$-$C_6$-Alkoxy, $C_1$-$C_6$-Halogenalkoxy substituiert ist.

12. Mittel zur Bekämpfung oder Verhütung eines Befalls durch Mikroorganismen, dadurch gekennzeichnet, dass es als mindestens eine aktive Komponente eine Verbindung der Formel I gemäss Anspruch 1 enthält.

13. Mittel gemäss Anspruch 12, dadurch gekennzeichnet, dass es als mindestens eine aktive Komponente eine Verbindung der Formel I gemäss Anspruch 2 enthält.

14. Mittel gemäss Anspruch 12, dadurch gekennzeichnet, dass es als mindestens eine aktive Komponente eine Verbindung der Formel I gemäss Anspruch 3 enthält.

15. Mittel gemäss Anspruch 12, dadurch gekennzeichnet, dass es als mindestens eine aktive Komponente eine Verbindung der Formel I gemäss einem der Ansprüche 4, 5 und 10 enthält.

16. Mittel gemäss Anspruch 12, dadurch gekennzeichnet, dass es als mindestens eine aktive Komponente eine Verbindung der Formel I gemäss einem der Ansprüche 6 bis 9 enthält.

17. Verfahren zur Bekämpfung oder Verhütung eines Befalls von Kulturpflanzen durch phytopathogene Mikroorganismen, dadurch gekennzeichnet, dass man eine gemäss Anspruch 1 definierte Verbindung der Formel I auf die Pflanze oder deren Standort oder auf Pflanzenteile appliziert.

18. Verfahren gemäss Anspruch 17, wobei die Pflanzenteile das Saatgut sind.

FO 7.5/PK/eg*

Europäisches Patentamt

# EUROPÄISCHER RECHERCHENBERICHT

0221014

Nummer der Anmeldung

EP 86810431.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 131 867 (BAYER AG)<br>* Ansprüche 1,4,5,6 *<br>-- | 1,11,<br>12,17 | C 07 D 239/26<br>C 07 D 241/12<br>A 01 N 43/54<br>A 01 N 43/60 |
| A | EP - A1 - 0 037 591 (BAYER AG)<br>* Ansprüche 1-4 *<br>-- | 1,11,<br>12,17 | |
| A | AU - B - 54 833/80 (ICI AUSTRALIA)<br>* Ansprüche 1,3,4,5 *<br>-- | 1,11,<br>12,17 | |
| A | US - A - 4 171 214 (BALASU-BRAMANIAN et al.)<br>* Zusammenfassung, Spalte 7, Zeile 20 - Spalte 9, Zeile 2*<br>---- | 1,11,<br>12,17 | |
| | | | RECHERCHIERTE SACHGEBIETE (Int. Cl.4) |
| | | | C 07 D 239/00<br>C 07 D 241/00 |

Der vorliegende Recherchenbericht wurde für alle Patentansprüche erstellt.

| Recherchenort | Abschlußdatum der Recherche | Prüfer |
|---|---|---|
| WIEN | 05-01-1987 | LUX |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82